# EUROPÄISCHE PATENTSCHRIFT

(11) **EP 1 830 835 B1**
(45) Veröffentlichungstag und Bekanntmachung des Hinweises auf die Patenterteilung: **14.03.2012**
(21) Anmeldenummer: 04802397.2
(22) Anmeldetag: 28.12.2004
(51) Int. Cl.: A61K 31/357, A61K 31/165, A61K 31/728, A61P 23/00

(54) **VERWENDUNG EINES VANILLOIDREZEPTORAGONISTEN ZUSAMMEN MIT EINEM GLYCOSAMINOGLYCAN ODER PROTEOGLYCAN F]R DIE HERSTELLUNG EINES MITTELS ZUR BEHANDLUNG VON GELENKSCHMERZEN UND VERFAHREN ZUR APPLIKATION DIESES MITTELS**
USE OF A VANILLOID RECEPTOR AGONIST TOGETHER WITH A GLYCOSAMINOGLYCAN OR PROTEOGLYCAN FOR PRODUCING AN AGENT FOR TREATING ARTICULAR PAINS AND METHOD FOR APPLYING SAID AGENT
UTILISATION D'UN AGONISTE DE RECEPTEUR VANILLOIDE AVEC UN GLUCOSAMINE GLYCANE OU PROTEOGLYCANE POUR LA PRODUCTION D'UNE SUBSTANCE DESTINEE A TRAITER LES DOULEURS ARTICULAIRES ET PROCEDE D'APPLICATION DE LADITE SUBSTANCE

(43) Veröffentlichungstag der Anmeldung: 12.09.2007
(73) Patentinhaber: Mestex AG, 8008 Zürich (CH)
(72) Erfinder: MEYER, Dominik, CH-8008 Zürich (CH)
(74) Vertreter: Lusuardi, Werther
(86) Internationale Anmeldenummer: PCT/CH2004/000757
(87) Internationale Veröffentlichungsnummer: WO 2006/069452

(56) Entgegenhaltungen:
- WO-A-2004/056305
- KARAI L ET AL: "Deletion of vanilloid receptor 1-expressing primary afferent neurons for pain control" JOURNAL OF CLINICAL INVESTIGATION, NEW YORK, NY, US, Bd. 113, Nr. 9, 2004, Seiten 1344-1352, XP002333034 ISSN: 0021-9738
- "Rote Liste", 1 January 2004 (2004-01-01), Rote Liste GmbH * 05-366 * * 05-372 *
- CROCHEMORE C ET AL: "Direct targeting of hippocampal neurons for apoptosis by glucocorticoids is reversible by mineralocorticoid receptor activation", MOLECULAR PSYCHIATRY, BASINGSTOKE, GB LNKD- DOI:10.1038/SJ.MP.4001679, vol. 10, no. 8, 1 August 2005 (2005-08-01) , pages 790-798, XP009125341, ISSN: 1359-4184
- "Rote Liste", 1 January 2004 (2004-01-01), Rote Liste GmbH * 05-366 * * 05-372 *
- CROCHEMORE C ET AL: "Direct targeting of hippocampal neurons for apoptosis by glucocorticoids is reversible by mineralocorticoid receptor activation", MOLECULAR PSYCHIATRY, BASINGSTOKE, GB LNKD- DOI:10.1038/SJ.MP.4001679, vol. 10, no. 8, 1 August 2005 (2005-08-01) , pages 790-798, XP009125341, ISSN: 1359-4184

## Beschreibung

Die Erfindung bezieht sich auf die Verwendung eines Vanilloidrezeptoragonisten zusammen mit einem Glycosaminoglycan oder Proteoglycan für die Herstellung eines Mittels zur Behandlung von Schmerzen gemäss dem Oberbegriff des Patentanspruchs 1.

Von Gelenken ausgehende Schmerzen haben ihren Ursprung häufig im Bereich der Gelenkkapsel oder im gelenknahen Bereich des Knochens. Dabei können viele Ätiologien in Frage kommen, z.B. arthrotische oder arthritische Krankheitsformen, mechanische oder andere Reizung der gelenknahen Knochenoberfläche, Reizung oder Verletzung der Gelenksbandstrukturen, Infekte, autoimmune Prozesse, u.s.w.. In allen Fällen, welche im Rahmen dieser Erfindung von Interesse sind, gehen die entstehenden Schmerzen von nociceptiven Nervenfasern im gelenknahen Bereich aus. Nociceptive Nervenfasern werden auch als C-Fasern und A-delta Fasern bezeichnet. Wird in ein so erkranktes Gelenk eine analgetische Substanz (z.B. Lokalanästhetika oder Morphine) injiziert, so werden die Beschwerden des Patienten gelindert. Allerdings haben die heute gebräuchlichen Substanzen eine nur beschränkte Wirkdauer, weshalb die Beschwerden meistens zurückkehren.

Zur Therapie schmerzhaft erkrankter Gelenke werden heute generell folgende Verfahren angewendet:
- Physiotherapie/Bewegungstherapie
- Systemische analgetische/antiphlogistische Therapie (etc.)
- Lokale analgetische/antiphiogistische Verfahren (etc.)
- Operative Verfahren:
- Arthroskopisch: Debridement, Gelenkstoilette, etc.
- Offen/Mini-offen: Gelenksersatz, Gelenkversteifung, etc.

In der Literatur wurden auch schon eine Reihe von bekannten Substanzen zur Therapie schmerzhafter, entzündlicher Gelenke vorgeschlagen, insbesondere:
- Osmiumsäure oder radioaktive Substanzen wie Technetium 99, welche zu einer Synoviorthese führen;
- Injektion von Lokalanästhetika, Hyaluronsäurepräparaten (etc.)
- Injektion von Antiphlogistika
- Injektion von Kontrastmitteln zur Gelenksdiagnostik
- Gelenkspülung zur Gelenkstoilette
- Chemische, thermische, elektrische oder chirurgische Ablation der gelenksversorgenden Nerven.

Alle bisher verwendeten Substanzen und Verfahren führen nur zu einer relativ kurzfristigen oder unvollständigen Schmerzfreiheit oder verursachen bleibende Schädigungen am Gelenk.

So besteht beispielsweise beim bekannten Verfahren der Synoviorthese der Nachteil der Zerstörung der molekularen Strukturen, insbesondere Denaturierung der Proteine, welche im Prozess der Arthritis und z.T. auch Arthroseentwicklung als Entzündungsauslöser wirken. Dabei entsteht eine Fibrose der Gelenkkapsel welche weniger entzündlich und somit auch weniger schmerzhaft ist. Gleichzeitig wird durch die bei der Synoviorthese auftretende Fibrose des Gelenkes die zumeist vorhandene und dabei ebenfalls zu behandelnde Hyperämie vermindert, woraus sich ebenfalls therapeutischer Nutzen ergibt. Die fibrotische Vemarbung nach Synoviorthese kann aber zu einer verminderten Beweglichkeit des Gelenkes führen, sowie zu einer verminderten Produktion von Synovialflüssigkeit und zur Zerstörung des Gelenkknorpels. Diese unerwünschte Fibrose der Gelenkskapsel sollte vermieden und nur die sensible Innervation des Gelenkes ausgeschaltet werden.

Ferner ist die Injektion von Capsaicin in das Gelenk zur Schmerztherapie bekannt. Capsaicin alleine brennt jedoch stark und erzeugt eine Gelenksentzündung und Knorpelschädigung.

Schliesslich ist aus der WO2006/06641 eine Mischung nach dem Oberbegriff des Anspruchs 1 bekannt, welche aber zwingenderweise eine nervenregenerationshemmende Substanz enthalten muss, bzw.
(i) im Falle einer intraartikulären Injektion von 9 ml einer Lösung von 500 nM Resiniferatoxin und 1% Hyaluronsäure nicht gleichzeitig 0,03mg Vincristin injiziert werden;
(ii) im Falle einer intraartikulären Injektion von 0,9 ml einer Lösung von 500 nM Resiniferatoxin und 1% Hyaluronsäure nicht gleichzeitig 0,03 mg Vincristin injiziert werden; und
(iii) im Falle einer intraartikulären Injektion von 9 ml einer Lösung von 500nM Resiniferatoxin und 1% Hyaluronsäure nicht gleichzeitig 0,5 mg/m2 Vincristin über 24 Stunden intravenös verabreicht wird.

Hier will die Erfindung Abhilfe schaffen. Der Erfindung liegt die Aufgabe zugrunde ein Mittel zur Behandlung von Gelenkschmerzen gemäss dem Oberbegriff des Anspruchs 1 bereitzustellen, welches eine verbesserte Schmerztherapie erlaubt.

Zwar wurde bereits die Anwendung von Vorläufersubstanzen (Glycosamin) von Glycosaminoglycanen in einem Gemisch mit 10-20 verschiedensten pflanzlichen Produkten (darunter auch Cayenne-Pfeffer und Capsaicin) in unterschiedlichen Konzentrationen zu Herstellung von Salben oder Paste zum Auftragen auf die Haut beschrieben, welche täglich aufgetragen werden muss. Die in diesem bekannten Gemisch enthaltenen Kräuter-Substanzen haben aber für den hier angestrebten Zweck eine schädliche Wirkung. Die Anwendung von Glycosaminen hat den weiteren entscheidenden Nachteil, dass diese Substanzen nicht für die Knorpelintegrität förderlich sind, ganz im Gegensatz beispielsweise zum Proteoglycan Hyaluronsäure. Im Übrigen kommen bei den obengenannten bekannten Anwendungen der zur oberflächlichen Applikation auf der Haut bestimmten Zubereitung Schmerzlinderungs-Mechanismen ähnlich der Elektro-Therapie mit Stimulation der Nerven-Enden zum Tragen, ohne dass dabei eine selektive Denervation der Schmerzfasern bewirkt würde, was das Ziel der Erfindung ist.

Die Erfindung löst die gestellte Aufgabe mit der Verwendung gemäss Anspruch 1.

Ueberraschenderweise wurde gefunden, dass die Nebenwirkungen von Vanilloidrezeptoragonisten, in Form des Resiniferatoxin (RTX) und seiner Salze (Entzündung und Schmerzen bei Injektion, Knorpelschaden) durch Beigabe von Glycosaminoglycanen oder Proteoglycanen, in Form der Hyaluronsäure sowie ihrer Salze ,deutlich reduziert werden und die erwünschten Wirkungen wie Schmerzbefreiung stärker erfolgen wurden als es die Substanzen getrennt erreichen würden. Diese Kombination erlaubt eine dosierte Freisetzung des Vanilloidrezeptoragonisten und schont gleichzeitig das Gelenk. Die Kombination hat durch die wieder stattfindende schmerzfrei Bewegung des Gelenkes einen knorpelaufbauenden Effekt welcher vor allem durch das Glycosaminoglycan in Form der Hyaluronsäure sowie ihrer Salze bewirkt wird.

Der Vanilloidrezeptoragonist in Form des Resiniferatoxin (RTX)und seiner Salze zusammen mit einem Glycosaminoglycan oder Proteoglycan in Form der Hyaluronsäure sowie ihrer Salze (aber ohne eine nervenregenerationshemmende Substanz) kann in ein schmerzhaftes oder erkranktes Gelenk des Körpers bei Mensch oder Tier lokal injiziert werden. Die injizierte Lösung kann entweder dort belassen werden oder nach einer gewissen Einwirkzeit wieder vollständig oder teilweise abgesogen werden. Das Glycosaminoglycan in Form der Hyaluronsäure sowie ihrer Salze bindet teilweise den Vanilloidrezeptoragonisten wodurch dieser langsamer freigesetzt wird und bei Einwirkung weniger Schmerzen entstehen. Ein weiterer Effekt ist, dass der Gelenkknorpel geschont und aufgebaut wird und weniger Entzündungsreaktion entsteht. Der Vanilloidrezeptoragonist diffundiert zu den sensiblen Nervenendigungen, weiche direkt oder indirekt den Bereich des Gelenkes innervieren, hemmt oder schädigt diese prädominant und führt damit zu einer verminderten Wahrnehmung der Gelenkschmerzen. Beide Substanzen haben zusammen jedoch einen synergistischen Effekt für die Schmerzfreiheit und Beweglichkeit des Gelenkes. Die Gelenkskapsel oder der Gelenkschmierbeutel kann dazu verwendet werden die Wirkung der Mischung auf den Ort der Schmerzentstehung zu konzentrieren und dadurch lokal eine höhere Konzentration und verlängerte Wirkdauer zu erlauben, als es ohne die schützende Gelenkskapsel oder den Gelenkschmierbeutel in der gleichen Konzentration und Verträglichkeit möglich wäre und gleichzeitig die Gefäss/Nervenstrukturen und andere Strukturen in der Nähe des Gelenkes verhältnismässig zu schonen. Somit wird eine langfristige Linderung der vom erkrankten Band-Kapsel-Gelenk-Komplex ausgehenden Schmerzempfindung durch Hemmung oder Ausschaltung der Reizleitung erlangt. Dieses Verfahren kann sowohl präventiv oder therapeutisch angewandt werden.

Die Vorteile der erfindungsgemässen Verwendung insbesondere bei einer lokalen Injektion der Mischung in die Gelenkkapsel oder in den Gelenkschmierbeutel sind die folgenden:
- Die intraartikuläre Injektion von Glycosaminoglycanen und Vanilloidrezeptoragonisten zur analgetischen Therapie von Gelenken führt zu einer weitgehenden Schonung der Kapsel-Bandstrukturen, der Synovia und der Knorpel-Knochenstrukturen und somit zur Erhaltung der physiologischen Verhältnisse.
- Die Nutzung der Gelenkskapsel als natürliche Grenze der Verteilung des Glycosaminoglycan und Vanilloidrezeptoragonisten.
- Das Verfahren ist durch Nicht-Spezialisten durchführbar.
- Das Verfahren ist mit einer dünnen, auch nicht-arthroskopischen Nadel durchführbar.
- Das Verfahren ist nicht infektionsgefährdend, im Gegensatz zum beliebten Verfahren der Cortisoninjektion, welches stark lokal infektionsfördernd ist, da Cortison lokal das Immunsystem hemmt.
- Das Verfahren führt zu einer sensiblen Denervation, d.h. einer Ausschaltung von schmerzleitenden Nerven.
- Erweiterung der Gelenksbeweglichkeit durch Aufhebung der schmerzhaften Bewegungseinschränkung im Gegensatz zur Synoviorthose, bei welcher durch die entstehende Kapselfibrose eine Bewegungseinschränkung erfolgt.
- Positive Vorbereitung für eine spätere Arthroplastik bei Anwendung an einem Gelenk. Durch die stimulierende Wirkung der wieder stattfindenden Belastung ohne Schmerz wird die Knochenbildung in der Extremität Insbesondere in Gelenknähe gefördert und der gelenknahe Knochen erhält eine für den späteren Halt einer Prothese vorteilhaftere Struktur.
- Keine lokale Fettgeweberesorption (Lipolyse)
- Keine Schwächung von kollagenen Sehnen/Band/Kapsel-Strukturen.
- Der Knorpel wird durch die schützende Wirkung von Glycosaminoglycanen geschont
- Durch die hochselektive Wirkung von Vanilloidrezeptoragonisten tritt keine motorische Beeinträchtigung ein, egal wo die Substanz gespritzt wird.
- Durch die hochselektive Wirkung des Vanilloidrezeptoragonisten tritt keine proprioceptive Beeinträchtigung ein, egal wo die Substanz gespritzt wird.
- Gegenüber der bekannten Verwendung von Glycosaminen, weisen die Glycosaminoglycane, in Form der Hyaluronsäure und ihrer Salze den Vorteil auf, dass sie Bestandteile der Gelenkflüssigkeit und des Knorpels sind und sowohl chemische wie auch mechanische Funktionen im Gelenk haben. Die Hyaluronsäure dämpft die Kontaktkräfte im Gelenk und hat eine schmierende, federnde und lubrifizierende Eigenschaft, welche die Glycosamine in ihrer Form nicht aufweisen. Ein weiterer essentieller Vorteil von Glycosaminoglycanen gegenüber Glycosaminen ist, dass Glycosaminoglycane nicht weiter verstoffwechselt werden im Gegensatz zu den Glycosaminen, welche wesentlich länger im Gelenk bleiben.

Die Erfindung wird im Folgenden für die Anwendung beim Menschen beschrieben, insbesondere beziehen sich die angegebenen Dosierungen auf die Humanapplikation. Die Erfindung eignet sich aber auch für den Veterinärbereich, wobei dort Anpassungen in der Dosierung vorgenommen werden müssen in Abhängigkeit vom Körpergewicht des jeweiligen Tieres.

Bei einer besonderen Ausführungsform der Erfindung werden dem Mittel weder
a) Kräuter oder andere zusätzliche pflanzliche Zusatzstoffe
b) trizyklische Antidepressiva
c) nicht-anästhetische Natriumkanal-Blocker
d) nicht-steroidale Antirheumatika; noch
e) Vasodilatator
beigemengt.

Durch das Weglassen der Kräutersubstanzen wird eine pharmakologisch nutzbare Reinheit erreicht, welche die Injektion zusammen mit einem Vanilloidrezeptoragonist in Form des Resiniferatoxin (RTX) in den Körper erleichtert und es werden keine allergischen oder pharmakologischen Nebenwirkungen erzeugt bei gleichzeitiger lokaler Denervation der Nervenfasern.

Vorzugsweise erfolgt die Schmerzbehandlung lokal und das Mittel ist insbesondere zur Behandlung folgender Indikationen vorgesehen:
a) Wundschmerzen nach OP in Form einer Spüllösung zur intraoperativen Anwendung bei einer offenen oder arthroskopischen oder endoskopischen Operation, inklusive Liposuction;
b) Gelenkschmerzen durch intraartikuläre Injektion bei
   Arthrose
   Rheumatoider Arthritis
   Infektiöser Arthritis
   Chondrocalzinose
   Ligamentären Schäden
   Meniskusläsion
   Knorpelschaden
   Synovitis
   Arthrofibrose
   Morbus Sudeck
   Nekrose der Gelenkanteile
   Neuropathischen Gelenkschmerzen
c) Knochenschmerzen nach Knochen-Operation durch Auftragung auf den Knochen, nach Beckenkammosteotomie oder Hallux-Valgus Korrektur;
d) Knochenschmerzen durch Injektion in den Knochen bei Femurkopfnekrose in den Femurkopf oder in den Wirbelkörper bei Osteochondrose;
e) Gelenksteife, insbesondere bei Arthrofibrose oder Frozen shoulder;
f) Muskelschmerzen, durch intramuskuläre Injektion, insbesondere bei Muskelfaserriss, Muskelkater oder spastischen Erkrankungen;
g) schmerzhaften Meniskus bei Meniskusdegeneration oder Meniskusriss;
h) Behandlung von Rückenschmerzen durch Injektion in die Bandscheibe bei Bandscheibendegeneration oder Bandscheibenriss
i) schmerzhafte Nerven, insbesondere Trigeminusneuralgie, Neurinom, MortonNeurom, Phantomschmerz oder Narbenneurom
j) Zahnschmerzen, insbesondere bei Karies, allen Formen von Zahnschmerz, Vor/bei/nach Zahnextraktion, Vor/bei/nach Zahnimplantat, lokale Anwendung bei Parodontitis, oder lokale Anwendung bei freiliegenden Zahnhälsen;
l) pleuritischen Beschwerden;
k) Darmbeschwerden, insbesondere bei Colitis ulcerosa, M.Crohn, Analfissur oder Hämorrhoiden.

Besonders vorteilhaft ist es das erfindungsgemäss hergestellte Mittel intraartikulär zu injizieren.

Bei einer weiteren Ausführungsform wird das Mittel in eine synoviale Höhle eingespritzt, welche nicht mit Urothel ausgekleidet ist. Es hat sich nämlich gezeigt, dass das Mittel eine ideale Wirkungsentfaltung auf synovialen Oberflächen zeigt, nicht jedoch auf dem Urothel wo noch Ethanol als Zusatz notwendig wäre zur Wirkungsentfaltung, diese Substanz ist jedoch im Gelenk sehr schädlich.

Erfindungsgemäss ist der Vanilloidrezeptoragonlst das Resiniferatoxin (RTX). Das Resiniferatoxin zeigt überraschenderweise ein optimales Wirkungsprofil und wesentlich weniger Nebenwirkungen, was sich überraschend deutlich in Kombination mit Hyaluronsäure als Proteoglycan zeigt.

Das Mittel sollte vorteilhafterweise eine Konzentration zwischen 10nanoMolar (nM) bis 10mMol aufweisen. Die für die erfindungsgemässe Verwendung angegebenen Konzentrationsbereiche sind dazu geeignet eine dauerhafte Denervation im Gelenk zu erzielen, wenn die Substanz injiziert wird. Durch die spezielle Kombination der Substanzen kann überraschenderweise ein Knorpelschaden wie auch eine Entzündung lokal verhindert werden, zusätzlich wird das brennende Gefühl bei Injektion reduziert. Ferner wird in dieser Erfindung ein geeignetes Medium beschrieben um die Substanzen zu injizieren. Bei geringerer lokaler Konzentration treten geringere Nebenwirkungen auf.

Der Vanilloidrezeptoragonist in Form des Resiniferatoxin (RTX) sollte vorteilhafterweise lokal eine Konzentration von 1nM bis 100µM, vorzugsweise von 100-2000nM. oder eine Dosis von 1ng-50µg/kg Körpergewicht aufweisen.

Das Glycosaminoglycan oder Proteoglycan ist aus folgender Gruppe ausgewählt:
eine Hyaluronsäure sowie ihre Salze davon. Das Molgewicht der Hyaluronsäure beträgt vorzugsweise 1000-5000kDa.

Die Dosierung des Mittels beträgt vorteilhafterweise 0,01 mg - 20g.

Das Mittel sollte ohne transdermale Trägerflüssigkeit verwendet wird. Transdermale Zubereitungen eignen sich nicht für die Injektion in den Körper und ausserdem soll die Zubereitung im behandelten Gelenk bleiben und nicht hinausdiffundieren.

Das Mittel wird vorteilhafterweise in einem geeigneten körperverträglichen Lösungsmittel lokal in die schmerzbefallene Gewebestruktur des Patienten injiziert oder lokal auf die Operationswunde geträufelt.

Bei einer besonderen Ausführungsform kann das Mittel zusätzlich ein Lokalanästhetikum enthalten. Dadurch entstehen weniger Schmerzen bei der Injektion. In geeigneter hoher Konzentration sind die Lokalanästhetika zusätzlich selbst neurotoxisch und unterstützen den gewünschten Effekt.

Bei einer weiteren Ausführungsform wird zusätzlich zum Glycosaminoglycan und Vanilloidrezeptoragonisten ein Röntgenkontrastmittel, z.B. ein Bariumzusatz oder ein MRI-Kontrastmittel verwendet, so dass eine bildgebende Kontrolle der Verteilung des Glycosaminoglycans und Vanilloidrezeptoragonisten im intrakapsulären Raum möglich ist und dadurch die Verteilung im Körper genau dokumentierbar ist. Als Kontrastmittel können je nach Verfahren folgende Substanzen verwendet werden:

| | |
|---|---|
| Röntgen, CT: | Jodhaltige Substanzen, z.B. trijodierte Benzoate oder lopamidol, idealerweise 30 - 80g/100ml oder z. B. 5 - 10% eines anderen Kontrastmittels, z.B. Barium. |
| MRI: | z.B. Gadolinium, z.B. pro 1ml: 469,01mg Gadopentat Dimeglumid, 0,99mg Meglumin, 0,4mg Diethylentriamin-pentaacetat. |

Bei einer weiteren Ausführungsform enthält das Mittel zusätzlich ein Steroid. Dadurch können Entzündungsreaktionen unterdrückt werden und ein synergistischer Effekt bezüglich Gelenkschmerz erzielt werden. Das Steroid kann z.B. Cortison sein. Beim allfälligen Auftreten einer entzündlichen Reaktion kann diese damit kontrolliert werden. Ausserdem kann man damit eine eher kausale Behandlung von schmerzhaften, entzündlichen Gelenkserkrankungen hinzufügen, welche die symptomatische, neurolytische Therapie unterstützt. Als besonders geeignet hat sich Betamethason erwiesen; z.B. 5 mg Betamethason als Diproprionat (kristalline Suspension) und 2 mg Betamethason als Dinatriumphosphat (Lösung in 1 ml, kann der total zu injizierenden Menge beigefügt werden). Diese Lösung ist äquivalent zu 45/23 mg Prednison/Prednisolon.

Bei einer weiteren Ausführungsform enthält das Mittel zusätzlich einen Vasokonstriktor, vorzugsweise Adrenalin, Noradrenalin, Phenylephrin oder Ornipressin, vorzugsweise alpha-adrenerge Vasokonstriktoren. Dadurch kann eine geringere systemische Verteilung und dadurch eine bessere systemische Verträglichkeit und bessere lokale Wirksamkeit erzielt werden. Mit Adrenalin kann die Gesamtdosis des Gemisches um zirka den Faktor 2 gesteigert werden, da so die systemische Wirkung durch die verminderte Resorption reduziert wird. Die Adrenalinkonzentration kann 1:10'000 bis 1:80'000 bis 1:200'000 betragen. Die Gesamtdosis an Adrenalin liegt bei 0,25 mg. Eine 50 ml Lösung von 1:200'000 Adrenalin enthält 0,25 mg Adrenalin.

Das Mittel wird vorteilhafterweise in einem körperverträglichen Lösungsmittel gelöst, vorzugsweise einem pharmakologisch akzeptablem Vehikel, insbesondere aus der Gruppe Natriumchlorid-Injektionslösung, Ringers Injektionslösung, isotonische Dextrose, steriles Wasser Dextroselösung, Laktierte Ringers Injektionslösung oder Mischungen davon. Dies ist essentiell falls die Substanzmischung injiziert oder die Körperregion damit gespült werden soll.

Bei einer weiteren Ausführungsform enthält das Mittel zusätzlich einen Permeationsförderer, vorzugsweise Dimethylsulfoxid, Ethoxyethylendiglycol, Ethanol, Phosphatidylcholine, Propylenglycol dipelargonate (DPPG), oder glycolysierte ethoxylierte Glyceride enthält. Dies ist insbesondere bei topischer Anwendung auf der Haut, aber auch zur besseren Permeation im Gewebe und durch Schleimhäute von Vorteil.

Bei einer weiteren Ausführungsform wird zusätzlich zum Glycosaminoglycan und Vanilloidrezeptoragonisten eine antibiotische, desinfizierende und/oder sterilisierende Substanz beigefügt.

Bei einer weiteren Ausführungsform enthält das Mittel zusätzlich ein Kalziumsalz. Dadurch wird die Wirksamkeit des Vanilloidrezeptoragonisten verbessert, da die Toxizität teilweise auf einer Erhöhung des intrazellulären Ca²⁺ Spiegels beruht. Die Kalziumionenkonzentration ist vorteilhafterweise grösser als 2mMol, vorzugsweise grösser als 4mMol,

Das Glycosaminoglycan macht vorteilhafterweise 0,5% - 10 %, vorzugsweise 1,0 %-3,0 % der Gesamtmischung aus. Bei diesem Konzentrationsbereich wird durch die chondroprotektive Wirkung des Glycosaminoglycans das brennende Gefühl bei der Injektion unterdrückt und das Gelenk geschont. Eine partielle Bindung des Vanilloidrezeptoragonisten an das Glycosaminoglycan kann eine verlangsamte Abgabe über einen verlängerten Zeitraum bewirken

Bei einer weiteren Ausführungsform ist das Mittel in einer Pufferlösung mit einem pH-Wert höher als 7,6 , vorzugsweise höher als 8,5 aufgelöst. Dadurch wird die brennende Schmerzhaftigkeit bei der Injektion verringert, da so die Rezeptor-lonenkanäle später geöffnet werden.

Bei einer anderen Ausführungsform ist das Mittel in einer Pufferlösung mit einem pH-Wert tiefer als 7,2, vorzugsweise tiefer als 6,5 aufgelöst. Dadurch wird die Wirkung des Vanilloidrezeptoragonisten vergrössert da sich so die Rezeptor-Ionen-Kanäle leichter und früher öffnen.

Das Mittel Ist vorzugsweise in einer geeigneten galenischen Zubereitung formuliert, welche eine verzögerte Freisetzung der Mischung gestattet. Dadurch kann die Wirkung mit weniger Nebenwirkungen optimiert werden.

Bei einer besonderen Ausführungsform enthält das Mittel eine Kombination von mehreren Glycosaminoglycanen oder Proteoglycanen. Dadurch erfolgt die Schmerzbehandlung noch effizienter und mit weniger Nebenwirkungen als nur mit einer Substanz allein. Das Glycosaminoglycan oder Proteoglycan kann in diesem Zusammenhang aus folgender Gruppe ausgewählt werden:
Keratan, Chondroitin Sulfat, Heparan Sulfat, N-Keratan Sulfat, O-Keratan Sulfat, Decorin, Perlecan, Dermatan Sulphat, Serglycin, Sydecan, Versican, Chondroitin Sulfat-A, Chondroitin Sulfat-B, Chondroitin Sulfat-C, Glycosamin Sulfat, eine Hyaluronsäure sowie ihre Salze davon.

Bei einer weiteren Ausführungsform enthält das Mittel eine Kombination von mehreren Vanilloidrezeptoragonisten. Da nicht alle Vanifloidrezeptaragonisten auf gleiche Weise an genau dieselben Rezeptoren andocken oder diese beeinflussen und nicht dasselbe Nebenwirkungsprofil haben, können Mischungen von solchen Vanilloiden vorteilhafte Synergien entwickeln. Die Vanilloidrezeptoragonisten, in diesem Zusammenhang können aus folgender Gruppe ausgewählt werden:
Ein Capsaicin, ein Vanilloid, Beta-aminoethyl-substituierte Phenyl-Alkanamide, Methylene substituierte-N-Phenylmethyl-Alkanamide, N-[(substituierte-Phenyl)methyl]-cis-monoungesättigte Alkenamide, Beta-Aminoethyl-substituierte Phenyl-Verbindungen, N-[(substituierte-Phenyl)methyl]doppelt-ungesättigte Amide, Anandamid, N-Oleoyldopamin, Trans-Capsaicin, Cis-Capsaicin, Civamide, SDZ-249-665, DA-5016, Arvanil, Dihydrocapsaicin, eine Resinifera-Verbindung, Isovelleral, Olvanil, Phorbol 12,13-didecanoate 20 homovanillate, Phorbol 12,13-dinonanoate 20-homovanillate, Tinyatoxin, sowie Salze der vorstehend genannten Verbindungen.

Das Mittel eignet sich insbesondere zur lokalen Behandlung von Sensationen welche von Nerven weitergeleitet werden welche Vanüloidrezeptoren tragen. Vanilloidrezeptoragonisten schädigen solche Nerven hoch selektiv.

Bei einer besonderen Ausführungsform weist der Vanilloidrezeptoragonist lokal eine Konzentration von 5nM bis 500µM, vorzugsweise von 100-5000nM oder eine Dosis von 1 ng-15mg/kg Körpergewicht, vorzugsweise von 10ng-50µg/kg Körpergewicht auf.

Bei einer weiteren Ausführungsform der Erfindung wird zusätzlich zum Glycosaminoglycan und Vanilloidrezeptoragonisten ein Vasokonstriktor verwendet.

Bei einer weiteren Ausführungsform der Erfindung wird zusätzlich zum Glycosaminoglycan und Vanilloidrezeptoragonisten eine antiphlogistisch wirkende Substanz verwendet, z.B. nicht-steroidale Antirheumatika, COX-2 Hemmer, Acetylsalycylsäure, u.s.w.

Bei einer weiteren Ausführungsform der Erfindung wird zusätzlich zur Substanzkombination Ca²⁺-Ionen oder vergleichbare Ionen in einer Konzentration höher als physiologisch vorhanden, im Lösungsmittel verwendet und gleichzeitig oder verzögert freigesetzt. Calcium ist für die Wirkung der Vanilloidrezeptoragonisten notwendig und verbessert deren Wirkung wenn in überphysiologischer Konzentration vorhanden. Die Konzentration an Calcium beträgt vorzugsweise >2mMol, insbesondere > 4mMol.

Bei einer weiteren Ausführungsform der Erfindung wird ein am Wirkort veränderter pH erzeugt, vorzugsweise durch Mischen des Vanilloidrezeptoragonisten mit einem geeigneten gepufferten Medium. Bei einer Verschiebung des pH kann ein verändertes Wirkprofil erzeugt werden. Bei einem pH kleiner als 7,4 ist die Wirkung des Vanilloidrezeptoragonisten verstärkt, bei einem pH grösser als 7,4 ist die Schmerzhaftigkeit der Injektion deutlich reduziert.

Bei einer weiteren Ausführungsform ist daher mittels geeigneten Puffermedien, welche auch verzögert freigesetzt werden können, durch Mikroenkapsulierung oder in fester Form, z.B. Pulver oder als Implantate wie Knochenersatzmaterial, der pH zuerst bei Applikation oder Injektion höher als 7,4 eingestellt und sinkt danach, vorzugsweise innerhalb von Minuten bis Stunden unter 7,4 ab.

Als Lösungsmedium kann anstelle von Glyzerin auch Wasser, Kochsalzlösung, Sodiumiothalamate, Iophendylat, Ricin, Poly-Ethlenglycol oder Propylenglykol verwendet werden. Der Vorteil von Glyzerin als Lösungsmittel ist, das dieses hyperbar und an sich auch schon etwas neurotoxisch ist.

Einige Stoffe haben sich als wirkungsverstärkend für die Substanzkombination erwiesen, so z.B. Calcium (insbesondere in einer Konzentration >2mM, vorzugsweise >4mM, Magnesium, Antioxidantien, Preservative und Excipienten, insbesondere Natriumbisulfit (> 0,2 %), NaHSO₃, Ammonium-Verbindungen, wie Ammoniumsulfat (NH₄)₂SO₄. 2 - 10 (-30%), Polysorbat 80 (PS80) ca. 0,025 mg/ml.

Das Glycosaminoglycan mit Vanilloidrezeptoragonist ist bevorzugt in einem körperverträglichen Lösungsmittel gelöst und wird zweckmässigerweise in einer Volumenmenge injiziert, welche dem verfügbaren Platz im zu behandelnden Gelenk entspricht, so dass dieses prallvoll wird. Damit wird der Vorteil einer optimalen lokalen Verteilung des Glycosaminoglycan mit Vanilloidrezeptoragonist erreicht. Es ist aber auch möglich weniger Flüssigkeit zu injizieren, dann ist es vorteilhaft das Gelenk gut zu bewegen zur besseren Verteilung des Glycosaminoglycan mit Vanilloidrezeptoragonist

Das in den intrakapsulären Bereich zu injizierende maximale Flüssigkeitsvolumen kann von 0,1 bis 150 ml betragen. Für ein Fingergelenk genügen etwa max. 1ml, für das Schultergelenk max. 10 ml, für das Kniegelenk max. etwa 30 - 50 ml, bevorzugt aber nicht über 2ml.

Die Dosierung der Substanzkombination hängt von der Lokalisation und Indikation ab.

Das Mittel wird vorteilhafterweise in einem geeigneten körperverträglichen Lösungsmittel lokal in die schmerzbefallene Gewebestruktur des Patienten injiziert oder lokal auf die Operationswunde geträufelt, an einen peripheren Nerven appliziert oder geeignet transcutan appliziert.

Bei einem bevorzugten Verfahren wird das Mittel in den intrakapsulären Bereich oder in den Gelenkschmierbeutel eines von Schmerzen betroffenen Gelenkes lokal injiziert. Das Mittel kann in einem körperverträglichen Lösungsmittel gelöst werden und davon vorzugsweise ein Flüssigkeitsvolumen von 0,1 bis 150 ml in den intrakapsulären Bereich oder in den Gelenkschmierbeutel des von Schmerzen betroffenen Gelenkes lokal injiziert werden. Die nociceptiven Nervenfasern werden dadurch für mindestens 14 Tage, vorzugsweise mindestens 8 Wochen schmerzunempfindlich gemacht.

Das Mittel wird vorzugsweise in einer solchen Konzentration verwendet, dass eine Neurolyse auftritt.

Das Mittel kann lokal, regional, systemisch (intravenös, peroral, subcutan, intramuskulär usw.) oder topisch auf der Haut oder Schleimhäuten angewendet werden.

Bei einem speziellen Verfahren wird der Vanilloidrezeptoragonist und das Glycosaminoglycan oder Proteoglycan simultan angewendet. Dadurch kann eine gute lokale Kontrolle und wenig Nebenwirkungen erzielt werden. Insbesondere wird eine gute örtlich und zeitlich synergistische Wirkung beider Substanzen erzielt.

Bei einem anderen Verfahren wird zuerst der Vanilloidrezeptoragonist und danach das Glycosaminoglycan oder Proteoglycan angewendet. Dadurch kann bei manchen Situationen die vulnerable Phase der Nervenregeneration besser genutzt werden und eine effizientere Wirkung erzielt werden.

Bei einem anderen Verfahren wird zuerst das Glycosaminoglycan oder Proteoglycan und danach der Vanilloidrezeptoragonist angewendet. Dadurch kann bei manchen Situationen die vulnerable Phase der Nervenregeneration besser genutzt werden und eine effizientere Wirkung erzielt werden.

Der Vanilloidrezeptoragonist und/oder die Glycosaminoglycan oder Proteoglycan können auch repetitiv angewendet werden. Dadurch kann bei manchen Situationen die vulnerable Phase der Nervenregeneration besser genutzt werden und eine effizientere Wirkung erzielt werden.

Schliesslich kann der Vanilloidrezeptoragonist und/oder das Glycosaminoglycan oder Proteoglycan mit verzögerter Freisetzung angewendet werden. Dadurch kann bei manchen Situationen die vulnerable Phase der Nervenregeneration besser genutzt werden und eine effizientere Wirkung erzielt werden. Es können so die Nebenwirkungen lokal und systemisch beider Substanzen entscheidend reduziert werden.

Bei einer besonderen Ausführungsform wird das zu behandelnde Gelenk vor Applikation des Mittels zur Verminderung von Schmerzen gekühlt. Dadurch entstehen bei der Injektion oder Applikation des Substanzgemisches weniger Schmerzen, da die Vanilloid-empfindlichen Ionenkanäle sich bei tieferer Temperatur weniger schnell öffnen.

Bei einer anderen Ausführungsform wird zusätzlich ein Lokalanästhetikum verwendet, entweder gleichzeitig mit dem Mittel oder vor Anwendung des Mittels. Dadurch wird erreicht, dass die Injektion oder Applikation nicht schmerzhaft ist. Das Lokalanästhetikum kann am selben Ort wie das Mittel oder entfernt davon injiziert werden. Dadurch können die lokalen Schmerzen bei der Injektion verringert werden.

Im Weiteren wird die Erfindung anhand zahlreicher Beispiele näher ausgeführt:

### Beispiel 1:

Der Therapeut brachte unter fakultativer, simultaner (Bildwandler, CT, Sonographie, MRI, etc.) oder nachträglicher (Röntgen, CT, MRI, Sonographie, Arthroskopie etc.) bildgebender Kontrolle eine Spritzennadel in den Gelenksraum eines Kniegelenkes und injizierte 9 ml einer Lösung von 500nM (ca.0.003mg) Resiniferatoxin mit 1% (ca. 90mg) Hyaluronsäure in den intrakapsulären Raum. Der Patient verspürte bereits 14 Stunden nach dem Eingriff deutliche Linderung seiner Beschwerden. Diese hielt für über 6 Monate an.

### Beispiel 2:

Die injizierte Lösung entsprach derjenigen von Beispiel 1 mit dem Unterschied, dass für das zu verwendende bildgebende Verfahren 5 ml eines sichtbaren Kontrastmittels (Iopamidol in einer Konzentration von 50g/100 ml) zugesetzt wurde, welches sich nach der Injektion innerhalb der Gelenkskapsel ausbreitete und so die Lage der Injektionsnadel und die Verteilung des 500nM (ca.0.003mg) Resiniferatoxin mit 1% (ca. 90mg) Hyaluronsäure innerhalb der Kapsel dokumentierte. Diese Lösung wurde unmittelbar nach erfolgter injektion wieder abgesogen. Sie könnte aber auch nach einer definierten substanzabhängigen Einwirkzeit oder gar nicht wieder abgesogen werden. Der Patient verspürte bereits 15 Stunden nach dem Eingriff deutliche Linderung seiner Beschwerden. Diese hielt für über 8 Monate an.

### Beispiel 3:

Der Therapeut legte einen dünnen Infusionskatheter analog zu einem Epiduralkatheter in das betroffene Gelenk ein und injizierte mit einem Perfusor eine Mischung von 2 Liter 100nM Resiniferatoxin mit 1% Hyaluronsäure in das betroffene Gelenk mit einer Rate von 1-10 ml/h während 12 h. Fakultativ legte er noch einen Abflusskatheter ein mit einem fakultativ definierten Abflusswiderstand (z.B. 20 mm Hg), um einen Flüssigkeitsumsatz zu erreichen. Mit dieser Methode erreichte der Therapeut eine gleichmässige Infiltration des schmerzhaften Gelenkes, ohne grosse Konzentrationsspitzen. Ausserdem konnte so die Einwirkzeit besser definiert werden. Bei einer nachfolgenden Arthroskopie nach 1, 2, 7, 14 und 28 d konnte gezeigt werden, dass nur sehr wenig entzündliches Gewebe vorhanden war. Der Patient verspürte bereits 12 Stunden nach dem Eingriff deutliche Linderung seiner Beschwerden. Diese hielt für über 1 Jahr an.

### Beispiel 4:

Nach Implantation einer Kniegelenkprothese injizierte der Therapeut 20 ml einer Mischung von 500nM (ca. 0.006mg) Resiniferatoxin mit 20 mg Hyaluronsäure in die wieder verschlossene Gelenkskapsel. Dadurch konnten die postoperativen Schmerzen minimiert werden.

### Beispiel 5:

Der Therapeut brachte unter fakultativer, simultaner (Bildwandler, CT, Sonographie, MRI, Arthroskopie etc.) oder nachträglicher (Röntgen, CT, MRI, Sonographie, etc.) bildgebender Kontrolle eine Spritzennadel in den Gelenksraum eines Kniegelenkes und injizierte 10 ml einer Lösung von 500µM (ca.0.003mg) Resiniferatoxin mit 60mg Hyaluronsäure gepuffert auf pH 8,6 in den intrakapsulären Raum. Der Patient verspürte bereits wenige Minuten nach dem Eingriff deutliche Linderung seiner Beschwerden. Diese hielt für über 6 Monate an.

### Beispiel 6:

Der Therapeut brachte nach vorheriger fakultativer lokaler Analgesie z.B. mit Lidocain unter fakultativer, simultaner (Bildwandler, CT, Sonographie, MRI, Arthroskopie etc.) oder nachträglicher (Röntgen, CT, MRI, Sonographie, etc.) bildgebender Kontrolle eine Spritzennadel in den Gelenksraum eines Kniegelenkes und injizierte 20 ml einer Lösung von 500nM (ca. 0.007mg) Resiniferatoxin mit 200mg Glycosamin Sulphat in physiologischer Kochsalzlösung in den intrakapsulären Raum. Der Patient verspürte bereits Minuten nach dem Eingriff deutliche Linderung seiner Beschwerden. Diese hielt für über 6 Monate an.

### Beispiel 7:

Der Therapeut brachte unter fakultativer, simultaner (Bildwandler, CT, Sonographie, MRI, Arthroskopie etc.) oder nachträglicher (Röntgen, CT, MRI, Sonographie, etc.) bildgebender Kontrolle eine Spritzennadel in den Gelenksraum eines Kniegelenkes und injizierte 20 ml einer Lösung von 0.007mg Resiniferatoxin mit 400mg Glycosamin Sulphat in physiologischer Kochsalzlösung in den intrakapsulären Raum. Der Patient verspürte bereits wenige Minuten nach dem Eingriff deutliche Linderung seiner Beschwerden. Diese hielt für über 6 Monate an.

### Beispiel 8:

Der Therapeut brachte nach fakultativer lokaler Analgesie unter fakultativer, simultaner (Bildwandler, CT, Sonographie, MRI, Arthroskopie etc.) oder nachträglicher (Röntgen, CT, MRI, Sonographie, etc.) bildgebender Kontrolle eine Spritzennadel in den Gelenksraum eines Kniegelenkes und injizierte 20 ml einer Lösung von 1 µM Tinyatoxin mit 800 mg Glycosamin Sulphat und 0.3mg Taxol in physiologischer Kochsalzlösung gepuffert auf pH 5 mit 8mM Ca(2+) in den intrakapsulären Raum. Der Patient verspürte bereits wenige Minuten nach dem Eingriff deutliche Linderung seiner Beschwerden. Diese hielt für über 8 Monate an.

### Beispiel 9:

Bei einem Patienten mit schmerzhafter Kapsulitis von Gelenken (z. B. "Frozen shoulder") wurde die eine Mischung von 500nM (ca. 0.003mg) Resiniferatoxin mit 20 mg Hyaluronsäure in physiologischer Kochsalzlösung in das Gelenk injiziert. Wiederum konnte die Verteilung der Substanz, bei Zusatz der entsprechenden Kontrastmittel bildgebend kontrolliert werden. Fakultativ wurde eine antiphlogistisch wirksame Substanz beigemischt. Wenige Minuten nach der Injektion liessen die Schmerzen dauerhaft nach, so dass der Patient mit Physiotherapie die durch die Kapsulitis verlorene Beweglichkeit wiedergewann. Bei dieser Anwendung ist manchmal lediglich eine vorübergehende Analgesie (2-3 Wochen) gewünscht, weshalb hier die Konzentration der neurotoxischen Substanz eher tief gehalten wurde

### Beispiel 10:

Bei einem Patienten mit schmerzhafter Kapsulitis von Gelenken wurde eine Mischung von 0.008 mg Resiniferatoxin mit 20mg Hyaluronsäure in physiologischer Kochsalzlösung in das Gelenk injiziert. Wenige Minuten nach der Injektion liessen die Schmerzen dauerhaft nach, so dass der Patient mit Physiotherapie die durch die Kapsulitis verlorene Beweglichkeit wiedergewann.

### Beispiel 11:

Der Therapeut injizierte in einen chronisch entzündeten Schleimbeutel (Bursa trochanterica) über den Trochanter major der Hüfte 5 ml einer 500nM (ca. 0.002mg) Resiniferatoxin mit 20 mg Hyaluronsäure in Glyzerin als Lösungsmittel. Innerhalb von 60 Minuten verschwanden die Beschwerden des Patienten, der mehrere Jahre an dieser Stelle beschwerdefrei blieb.

### Beispiel 12:

Der Therapeut injizierte 0.5 ml einer Lösung bestehend aus 500nM (ca. 0.0001mg) Resiniferatoxin mit 5 mg Hyaluronsäure sowie 5 % Kontrastmittel in physiologischer Kochsalzlösung als Lösungsmittel in ein schmerzhaftes, arthrotisches Fingergelenk. Nach circa 15 Minuten verschwanden die Beschwerden des Patienten für mehrere Monate. Die korrekte Position der Injektionsnadel konnte mittels des Kontrastmittels dokumentiert werden.

## Patentansprüche

1. Verwendung eines Vanilloidrezeptoragonisten zusammen mit einem Glycosaminoglycan oder Proteoglycan für die Herstellung eines Mittels zur Behandlung von Schmerzen, wobei
A) der Vanilloidrezeptoragonist das Resiniferatoxin (RTX) sowie seine Salze ist; und
B) das Glycosaminoglycan oder Proteoglycan aus folgender Gruppe ausgewählt ist: Hyaluronsäure sowie Salze davon;
**dadurch gekennzeichnet, dass**
(i) im Falle einer intraartikulären Injektion von 9 ml einer Lösung von 500 nM Resiniferatoxin und 1% Hyaluronsäure nicht gleichzeitig 0,03mg Vincristin injiziert werden;
(ii) im Falle einer intraartikulären Injektion von 0,9 ml einer Lösung von 500 nM Resiniferatoxin und 1% Hyaluronsäure nicht gleichzeitig 0,03 mg Vincristin injiziert werden; und
(iii) im Falle einer intraartikulären Injektion von 9 ml einer Lösung von 500nM Resiniferatoxin und 1 % Hyaluronsäure nicht gleichzeitig 0,5 mg/m2 Vincristin über 24 Stunden intravenös verabreicht wird.

2. Verwendung nach Anspruch 1, **dadurch gekennzeichnet, dass** keine Kräuter oder andere zusätzliche pflanzliche Zusatzstoffe dem Mittel beigemengt werden.

3. Verwendung nach Anspruch 1 oder 2, **dadurch gekennzeichnet, dass** kein trizyklisches Antidepressivum mitverwendet wird.

4. Verwendung nach einem der Ansprüche 1 bis 3, **dadurch gekennzeichnet, dass** kein nicht-anästhetischer Natriumkanal-Blocker mitverwendet wird.

5. Verwendung nach einem der Ansprüche 1 bis 4, **dadurch gekennzeichnet, dass** kein nicht-steroidales Antirheumatikum mitverwendet wird.

6. Verwendung nach einem der Ansprüche 1 bis 5, **dadurch gekennzeichnet, dass** kein Vasodilatator mitverwendet wird.

7. Verwendung nach einem der Ansprüche 1 bis 6, **dadurch gekennzeichnet, dass** die Schmerzbehandlung lokal erfolgt.

8. Verwendung nach einem der Ansprüche 1 bis 7, **dadurch gekennzeichnet, dass** das Mittel zur Behandlung folgender Indikationen vorgesehen ist:
a) Wundschmerzen nach OP in Form einer Spüllösung zur intraoperativen Anwendung bei einer offenen oder arthroskopischen oder endoskopischen Operation, inklusive Liposuction;
b) Gelenkschmerzen durch intraartikuläre Injektion bei
Arthrose
Rheumatoider Arthritis
infektiöser Arthritis
Chondrocalzinose
Ligamentären Schäden
Meniskusläsion
Knorpelschaden
Synovitis
Arthrofibrose
Morbus Sudeck
Nekrose der Gelenkanteile
Neuropathischen Gelenkschmerzen
c) Knochenschmerzen nach Knochen-Operation durch Auftragung auf den Knochen, nach Beckenkammosteotomie oder Hallux-Valgus Korrektur;
d) Knochenschmerzen durch Injektion in den Knochen bei Femurkopfnekrose in den Femurkopf oder in den Wirbelkörper bei Osteochondrose;
e) Gelenksteife, insbesondere bei Arthrofibrose oder Frozen shoulder;
f) Muskelschmerzen, durch intramuskuläre Injektion, insbesondere bei Muskelfaserriss, Muskelkater oder spastischen Erkrankungen;
g) schmerzhaften Meniskus bei Meniskusdegeneration oder Meniskusriss;
h) Behandlung von Rückenschmerzen durch Injektion in die Bandscheibe bei Bandscheibendegeneration oder Bandscheibenriss
i) schmerzhafte Nerven, insbesondere Trigeminusneuralgie, Neurinom, MortonNeurom, Phantomschmerz oder Narbenneurom
j) Zahnschmerzen, insbesondere bei Karies, allen Formen von Zahnschmerz, Vor/bei/nach Zahnextraktion, Vor/bei/nach Zahnimplantat, lokale Anwendung bei Parodontitis, oder lokale Anwendung bei freiliegenden Zahnhälsen;
l) pleuritischen Beschwerden;
k) Darmbeschwerden, insbesondere bei Colitis ulcerosa, M.Crohn, Analfissur oder Hämorrhoiden.

9. Verwendung nach Anspruch einem der Ansprüche 1 bis 8, **dadurch gekennzeichnet, dass** das Mittel intraartikulär injiziert wird.

10. Verwendung nach Anspruch einem der Ansprüche 1 bis 9, **dadurch gekennzeichnet, dass** das Mittel in eine synoviale Höhle eingespritzt wird, welche nicht mit Urothel ausgekleidet ist.

11. Verwendung nach einem der Ansprüche 1 bis 10, **dadurch gekennzeichnet, dass** das Mittel eine Konzentration zwischen 10nanoMolar (nM) bis 10mMol aufweist.

12. Verwendung nach Anspruch 11, **dadurch gekennzeichnet, dass** der Vanilloidrezeptoragonist eine Konzentration von 1nM bis 100µM, vorzugsweise von 100-2000nM, Dosis von 1ng-50µg/kg Körpergewicht aufweist.

13. Verwendung nach Anspruch 1 **dadurch gekennzeichnet, dass** die Hyaluronsäure ein Molgewicht von 1000-5000kDa aufweist.

14. Verwendung nach einem der Ansprüche 1 bis 13, **dadurch gekennzeichnet, dass** die Dosierung des Mittels 0,01mg - 20g beträgt.

15. Verwendung nach einem der Ansprüche 1 bis 14, **dadurch gekennzeichnet, dass** das Mittel ohne transdermale Trägerflüssigkeit verwendet wird.

16. Verwendung nach einem der Ansprüche 1 bis 15, **dadurch gekennzeichnet, dass** das Mittel in einem geeigneten körperverträglichen Lösungsmittel lokal in die schmerzbefallene Gewebestruktur des Patienten injiziert wird oder lokal auf die Operationswunde geträufelt wird.

17. Verwendung nach einem der Ansprüche 1 bis 16, **dadurch gekennzeichnet, dass** das Mittel zusätzlich ein Lokalanästhetikum enthält.

18. Verwendung nach einem der Ansprüche 1 bis 17, **dadurch gekennzeichnet, dass** das Mittel zusätzlich ein Röntgenkontrastmittel enthält, vorzugsweise in Form von gadoliniumhaltigen, jodhaltigen oder bariumhaltige Substanzen.

19. Verwendung nach einem der Ansprüche 1 bis 18, **dadurch gekennzeichnet, dass** das Mittel zusätzlich ein Steroid enthält.

20. Verwendung nach einem der Ansprüche 1 bis 19, **dadurch gekennzeichnet, dass** das Mittel zusätzlich einen Vasokonstriktor enthält, vorzugsweise Adrenalin, Noradrenalin, Phenylephrin oder Ornipressin.

21. Verwendung nach einem der Ansprüche 1 bis 20, **dadurch gekennzeichnet, dass** das Mittel in einem körperverträglichen Lösungsmittel gelöst ist, vorzugsweise einem pharmakologisch akzeptablem Vehikel, insbesondere aus der Gruppe Natriumchlorid-Injektionslösung, Ringiers Injektionslösung, isotonische Dextrose, steriles Wasser Dextroselösung, Laktierte Ringers Injektionslösung oder Mischungen davon.

22. Verwendung nach einem der Ansprüche 1 bis 21, **dadurch gekennzeichnet, dass** das Mittel zusätzlich einen Permeationsförderer, vorzugsweise Dimethylsulfoxid, Ethoxyethylendiglycol, Ethanol, Phosphatidylcholine, Propylenglycol dipelargonate (DPPG), oder glycolysierte ethoxylierte Glyceride enthält.

23. Verwendung nach einem der Ansprüche 1 bis 22, **dadurch gekennzeichnet, dass** das Mittel zusätzlich ein Kalziumsalz enthält.

24. Verwendung nach Anspruch 23, **dadurch gekennzeichnet, dass** die Kalziumionenkonzentration grösser als 2mMol, vorzugsweise grösser als 4mMol beträgt.

25. Verwendung nach einem der Ansprüche 1 bis 24, **dadurch gekennzeichnet, dass** das Glycosaminoglycan 0,5% - 10 %, vorzugsweise 1,0 %- 3,0 % der Gesamtmischung ausmacht.

26. Verwendung nach einem der Ansprüche 1 bis 25, **dadurch gekennzeichnet, dass** das Mittel ein einer Pufferlösung mit einem pH-Wert höher als 7,6, vorzugsweise höher als 8,5 aufgelöst ist.

27. Verwendung nach einem der Ansprüche 1 bis 25, **dadurch gekennzeichnet, dass** das Mittel in einer Pufferlösung mit einem pH-Wert tiefer als 7,2, vorzugsweise tiefer als 6,5 aufgelöst ist.

28. Verwendung nach einem der Ansprüche 1 bis 27, **dadurch gekennzeichnet, dass** das Mittel in einer geeigneten galenischen Zubereitung formuliert ist, welche eine verzögerte Freisetzung der Mischung gestattet.

29. Verwendung nach einem der Ansprüche 1 bis 28, **dadurch gekennzeichnet, dass** das Mittel eine Kombination von mehreren Glycosaminoglycanen oder Proteoglycanen enthält.

30. Verwendung nach einem der Ansprüche 1 bis 29, **dadurch gekennzeichnet, dass** das Mittel eine Kombination von mehreren Vanilioidrezeptoragonisten enthält.

31. Verwendung nach einem der Ansprüche 1 bis 30 für die Herstellung eines Mittels zur lokalen Behandlung von Sensationen welche von Nerven weitergeleitet werden welche Vanilloidrezeptoren tragen.

32. Verwendung nach Anspruch 1, **dadurch gekennzeichnet, dass** der Vanilloidrezeptoragonist lokal eine Konzentration von 5nM bis 500µM, vorzugsweise von 100-5000nM oder eine Dosis von 1 ng-15mg/kg Körpergewicht, vorzugsweise von 10ng-50µg/kg Körpergewicht aufweist.

33. Verwendung nach einem der Ansprüche 1 bis 32, **dadurch gekennzeichnet, dass** das Mittel in einem geeigneten körperverträglichen Lösungsmittel lokal in die schmerzbefallene Gewebestruktur des Patienten injiziert wird oder lokal auf die Operationswunde geträufelt wird, an einen peripheren Nerven appliziert wird oder geeignet transcutan appliziert wird.

## Claims

1. A use of a vanilloid receptor agonist together with a glycosaminoglycan or proteoglycan for producing an agent for treating pain, wherein
A) the vanilloid receptor agonist is the resiniferatoxin (RTX) and the salts thereof; and
B) the glycosaminoglycan or proteoglycan is selected from the following group:
Hyaluronic acid and salts thereof;
**characterized in that**
(i) in the case of an intra-articular injection of 9 ml of a solution of 500 nM of resiniferatoxin and 1% hyaluronic acid, 0.03 mg of vincristine is not injected at the same time;
(ii) in the case of an intra-articular injection of 0.9 ml of a solution of 500 nM of resiniferatoxin and 1% hyaluronic acid, 0.03 mg of vincristine is not injected at the same time; and
(iii) in the case of an intra-articular injection of 9 ml of a solution of 500 nM of resiniferatoxin and 1% hyaluronic acid, 0.5 mg/m2 of vincristine is not administered intravenously over 24 hours at the same time.

2. The use according to claim 1, **characterized in that** no herbs or other additional herbal additives are added to the agent.

3. The use according to claim 1 or claim 2, **characterized in that** no tricyclic antidepressant is used at the same time.

4. The use according to any one of the claims 1 to 3, **characterized in that** no non-anaesthetic sodium channel blockers are used at the same time.

5. The use according to any one of the claims 1 to 4, **characterized in that** no non-steroidal antirheumatic agent is used at the same time.

6. The use according to any one of the claims 1 to 5, **characterized in that** no vasodilator is used at the same time.

7. The use according to any one of the claims 1 to 6, **characterized in that** the pain treatment is carried out locally.

8. The use according to any one of the claims 1 to 7, **characterized in that** the agent is provided for treating the following indications:
a) post-surgery wound pain, in the form of a rinsing solution for intraoperative use during an open or arthroscopic or endoscopic surgery, inclusive liposuction;
b) arthralgia, by intra-articular injection with
arthrosis
rheumatoid arthritis
infectious arthritis
chondrocalcinosis
ligamentous damages
meniscal lesion
cartilage damage
synovitis
arthrofibrosis
Morbus Sudeck
necrosis of the joint sections
neuropathic joint pain
c) bone pain after bone surgery, by applying onto the bone, after iliac crest osteotomy or hallux valgus correction;
d) bone pain, by injection into the bone, in the case of femoral head necrosis into the femoral head, or into the vertebral body in the case of osteochondrosis;
e) joint stiffness, in particular in the case of arthrofibrosis or frozen shoulder;
f) muscle pains, by intramuscular injection, in particular in the case of a torn muscle fiber, muscle soreness or spastic diseases;
g) meniscal pain in the case of meniscal degeneration or a torn meniscus;
h) treatment of backache by injection into the intervertebral disk in the case of intervertebral disk degeneration or herniated disk;
i) painful nerves, in particular trigeminal neuralgia, neurinoma, Morton's neuroma, phantom pain or scar neuroma;
j) toothaches, in particular in the case of caries, all forms of toothaches, before/during/after tooth extraction, before/during/after tooth implantation, local application in the case of periodentitis, or local application in the case of exposed tooth necks;
l) pleuritic disorders;
k) intestinal disorders, in particular in the case of ulcerative colitis, M. Crohn, anal fissure or hemorrhoids.

9. The use according to any one of the claims 1 to 8, **characterized in that** the agent is injected intra-articularly.

10. The use according to any one of the claims 1 to 9, **characterized in that** the agent is injected into a synovial cavity which is not lined with urothelium.

11. The use according to any one of the claims 1 to 10, **characterized in that** the agent has a concentration between 10 nanoMolar (nM) and 10 mmol.

12. The use according to claim 11, **characterized in that** the vanilloid receptor agonist has a concentration of 1 nM to 100 µm, preferably of 100 - 2000 nM, or a dose of 1 ng - 50 µg/kg of body weight.

13. The use according to claim 1, **characterized in that** the hyaluronic acid has a molar weight of 1000 - 5000 kDa.

14. The use according to any one of the claims 1 to 13, **characterized in that** the dosage of the agent is 0.01 mg - 20 g.

15. The use according to any one of the claims 1 to 14, **characterized in that** the agent is used without transdermal carrier fluid.

16. The use according to any one of the claims 1 to 15, **characterized in that** the agent is injected in a suitable biocompatible solvent locally into the pain-affected tissue structure of the patient or is locally dripped onto the surgical wound.

17. The use according to any one of the claims 1 to 16, **characterized in that** the agent additionally contains a local anaesthetic.

18. The use according to any one of the claims 1 to 17, **characterized in that** the agent additionally contains an X-ray contrast medium, preferably in the form of gadolinium-containing, iodine-containing or barium-containing substances.

19. The use according to any one of the claims 1 to 18, **characterized in that** the agent additionally contains a steroid.

20. The use according to any one of the claims 1 to 19, **characterized in that** the agent additionally contains a vasoconstrictor, preferably adrenalin, noradrenalin, phenylephrine or ornipressin.

21. The use according to any one of the claims 1 to 20, **characterized in that** the agent is dissolved in a biocompatible solvent, preferably a pharmacologically acceptable excipient, in particular from the group comprising sodium chloride injection solution, Ringer's injection solution, isotonic dextrose, sterile water, dextrose solution, lactated Ringer's injection solution, or mixtures thereof.

22. The use according to any one of the claims 1 to 21, **characterized in that** the agent additionally contains a permeation enhancer, preferably dimethylsulfoxid, ethoxyethylendiglycol, ethanol, phosphatdycholine, propylenglycol dipelargonate (DPPG), or glycolized ethoxylated glycerides.

23. The use according to any one of the claims 1 to 22, **characterized in that** the agent additionally contains a calcium salt.

24. The use according to claim 23, **characterized in that** the calcium ion concentration is greater than 2 mmol, preferably greater than 4 mmol.

25. The use according to any one of the claims 1 to 24, **characterized in that** the glycosaminoglycan accounts for 0.5% - 10%, preferably 1.0% - 3% of the total mixture.

26. The use according to any one of the claims 1 to 25, **characterized in that** the agent is dissolved in a buffer solution having a pH value higher than 7.6, preferably higher than 8.5.

27. The use according to any one of the claims 1 to 25, **characterized in that** the agent is dissolved in a buffer solution having a pH value lower than 7.2, preferably lower than 6.5.

28. The use according to any one of the claims 1 to 27, **characterized in that** the agent is formulated in a suitable galenic preparation which enables a delayed release of the mixture.

29. The use according to any one of the claims 1 to 28, **characterized in that** the agent contains a combination of a plurality of glycosaminoglycans or proteoglycans.

30. The use according to any one of the claims 1 to 29, **characterized in that** the agent contains a combination of a plurality of vanilloid receptor agonists.

31. The use according to any one of the claims 1 to 30, for the production of an agent for local treatment of sensations which are transmitted by nerves that carry vanilloid receptors.

32. The use according to claim 1, **characterized in that** the vanilloid receptor agonist has a concentration locally of 5 nM to 500 µM, preferably of 100 - 5000 nM, or a dose of 1 ng - 15 mg/kg of body weight, preferably of 10 ng - 50 µg/kg of body weight.

33. The use according to any one of the claims 1 to 32, **characterized in that** the agent is injected in a suitable biocompatible solvent locally into the pain-affected tissue structure of the patient or locally dripped onto the surgical wound, or applied to a peripheral nerve, or applied transcutaneously in a suitable manner.

## Revendications

1. Utilisation d'un agoniste de récepteur vanilloïde avec un glucosamine glycane ou protéoglycane pour la production d'une substance destinée à traiter des douleurs,
A) l'agoniste de récepteur vanilloïde étant la résinifératoxine (RTX) ainsi que ses sels ; et
B) le glucosamine glycane ou le protéoglycane étant sélectionné dans le groupe suivant : acide hyaluronique ainsi que ses sels ;
**caractérisé en ce que**
(i) en cas d'injection intra-articulaire de 9 mL d'une solution de 500 nM de résinifératoxine et de 1 % d'acide hyaluronique, 0,03 mg de vincristine ne sont pas injectés de façon simultanée ;
(ii) en cas d'injection intra-articulaire de 0,9 mL d'une solution de 500 nM de résinifératoxine et de 1 % d'acide hyaluronique, 0,03 mg de vincristine ne sont pas injectés de façon simultanée ;
(iii) en cas d'injection intra-articulaire de 9 mL d'une solution de 500 nM de résinifératoxine et de 1 % d'acide hyaluronique, 0,5 mg/m2 de vincristine ne sont pas administrés simultanément en 24 heures par voie intraveineuse.

2. Utilisation selon la revendication 1, **caractérisée en ce qu'**aucune herbe ni autre additif végétal supplémentaire ne sont mélangés à la substance.

3. Utilisation selon la revendication 1 ou 2, **caractérisée en ce qu'**aucun antidépresseur tricyclique n'est utilisé conjointement.

4. Utilisation selon l'une des revendications 1 à 3, **caractérisée en ce qu'**aucun bloqueur des canaux sodiques non anesthésiant n'est utilisé conjointement.

5. Utilisation selon l'une des revendications 1 à 4, **caractérisée en ce qu'**aucun antirhumatismal non stéroïdien n'est utilisé conjointement.

6. Utilisation selon l'une des revendications 1 à 5, **caractérisée en ce qu'**aucun vasodilatateur n'est utilisé conjointement.

7. Utilisation selon l'une des revendications 1 à 6, **caractérisée en ce que** le traitement antidouleur est effectué localement.

8. Utilisation selon l'une des revendications 1 à 7, **caractérisée en ce que** la substance est prévue pour le traitement des indications suivantes :
a) douleurs d'une plaie à la suite d'une opération sous forme de solution de rinçage pour l'utilisation peropératoire lors d'une opération ouverte, ou arthroscopique ou endoscopique, y compris la liposuccion ;
b) douleurs articulaires par injection intra-articulaire dans les cas suivants:
arthrose
arthrite rhumatismale
arthrite infectieuse
chondrocalcinose
dommages ligamentaires
lésion du ménisque
dommage du cartilage
synovite
arthrofibrose
maladie de Sudeck
nécrose de segments articulaires
douleurs articulaires névropathiques
c) douleurs osseuses à la suite d'une opération osseuse par application sur l'os, à la suite d'une ostéotomie de la crête iliaque ou d'une correction de l'hallux-valgus ;
d) douleurs osseuses par injection dans l'os en cas de nécrose de la tête du fémur dans la tête du fémur ou dans le corps vertébral en cas d'ostéochondrose ;
e) ankylose, en particulier en cas d'arthrofibrose ou d'épaule bloquée ;
f) douleurs musculaires, par injection intramusculaire, en particulier en cas de déchirure de fibres musculaires, de raideur après l'effort ou d'affections spasmodiques ;
g) ménisque douloureux en cas de dégénérescence du ménisque ou de fracture du ménisque ;
h) traitement de douleurs dorsales par injection dans le disque intervertébral en cas de dégénérescence ou en cas de dégénérescence du disque intervertébral ou de fracture du disque intervertébral
i) nerfs douloureux, en particulier névralgie essentielle du trijumeau, névrome, névrome de Morton, algo-hallucinose ou névrome cicatriciel ;
j) douleurs dentaires, en particulier en cas de caries, de toutes formes de douleur dentaire, avant/pendant/après une extraction dentaire, avant/pendant/après une pose d'implant dentaire, utilisation locale en cas de parodontite, ou utilisation locale en cas de collet dentaire dénudé ;
l) douleurs pleurétiques ;
k) douleurs intestinales, en particulier en cas de colite ulcéreuse, maladie de Crohn, fissure anale ou hémorroïdes.

9. Utilisation selon l'une des revendications 1 à 8, **caractérisée en ce que** la substance est injectée par voie intra-articulaire.

10. Utilisation selon l'une des revendications 1 à 9, **caractérisée en ce que** la substance est injectée dans une cavité synoviale sans revêtement urothélial.

11. Utilisation selon l'une des revendications 1 à 10, **caractérisée en ce que** la substance présente une concentration entre 10 nanomolaire (nM) et 10 mMol.

12. Utilisation selon la revendication 11, **caractérisée en ce que** l'agoniste de récepteur vanilloïde présente une concentration de 1 nM à 100 µM, de préférence de 100 - 2000 nM, dose de 1 ng-50 µg/kg de poids corporel.

13. Utilisation selon la revendication 1, **caractérisée en ce que** l'acide hyaluronique présente une masse molaire de 1000 - 5000 kDa.

14. Utilisation selon l'une des revendications 1 à 13, **caractérisée en ce que** la posologie de la substance est de 0,01 mg - 20 g.

15. Utilisation selon l'une des revendications 1 à 14, **caractérisée en ce que** la substance est utilisée sans liquide vecteur transdermique.

16. Utilisation selon l'une des revendications 1 à 15, **caractérisée en ce que** la substance est injectée, dans un solvant approprié pouvant être supporté par le corps, localement dans la structure tissulaire du patient affectée par la douleur ou bien est instillée localement sur la plaie opératoire.

17. Utilisation selon l'une des revendications 1 à 16, **caractérisée en ce que** la substance contient en plus un anesthésiant local.

18. Utilisation selon l'une des revendications 1 à 17, **caractérisée en ce que** la substance contient en plus un produit radio-opaque, de préférence sous forme de substances contenant du gadolinium, de l'iode ou du baryum.

19. Utilisation selon l'une des revendications 1 à 18, **caractérisée en ce que** la substance contient en plus un stéroïde.

20. Utilisation selon l'une des revendications 1 à 19, **caractérisée en ce que** la substance contient en plus un vasoconstricteur, de préférence de l'adrénaline, de la noradrénaline, de la phényléphrine ou de l'ornipressine.

21. Utilisation selon l'une des revendications 1 à 20, **caractérisée en ce que** la substance est diluée dans un solvant pouvant être supporté par le corps, de préférence un véhicule acceptable au plan pharmaceutique, en particulier issu du groupe solution d'injection de chlorure de sodium, solution d'injection de Ringer, dextrose isotonique, solution aqueuse stérile, solution de dextrose, solution d'injection de Ringer lactate ou des mélanges de celles-ci.

22. Utilisation selon l'une des revendications 1 à 21, **caractérisée en ce que** la substance contient en plus un activateur de perméation, de préférence diméthylsulfoxyde, éthoxyéthylène diglycol, éthanol, phosphatidylcholine, dipélargonate de propylèneglycol (DPPG), ou des glycérides éthoxylés glycolysés.

23. Utilisation selon l'une des revendications 1 à 22, **caractérisée en ce que** la substance contient en plus un sel de calcium.

24. Utilisation selon la revendication 23, **caractérisée en ce que** la concentration d'ions calcium est supérieure à 2 mMol, de préférence supérieure à 4 mMol.

25. Utilisation selon l'une des revendications 1 à 24, **caractérisée en ce que** le glucosamine glycane représente 0,5 % - 10 %, de préférence 1,0 % - 3,0 %, du mélange total.

26. Utilisation selon l'une des revendications 1 à 25, **caractérisée en ce que** la substance est diluée dans une solution tampon ayant un pH supérieur à 7,6, de préférence supérieur à 8,5.

27. Utilisation selon l'une des revendications 1 à 25, **caractérisée en ce que** la substance est diluée dans une solution tampon ayant un pH inférieur à 7,2, de préférence inférieur à 6,5.

28. Utilisation selon l'une des revendications 1 à 27, **caractérisée en ce que** la substance est formulée dans une préparation galénique appropriée qui permet une libération retardée du mélange.

29. Utilisation selon l'une des revendications 1 à 28, **caractérisée en ce que** la substance contient une combinaison de plusieurs glucosamines glycanes ou protéoglycanes.

30. Utilisation selon l'une des revendications 1 à 29, **caractérisée en ce que** la substance présente une combinaison de plusieurs agonistes de récepteur vanilloïde.

31. Utilisation selon l'une des revendications 1 à 30 pour le traitement local de sensations qui sont transmises par les nerfs qui portent des récepteurs vanilloïdes.

32. Utilisation selon la revendication 1, **caractérisée en ce que** l'agoniste de récepteur vanilloïde présente localement une concentration de 5 nM à 500 µM, de préférence de 100 - 5000 nM ou une dose de 1 ng - 15 mg/kg de poids corporel, de préférence de 10 ng - 50 µg/kg de poids corporel.

33. Utilisation selon l'une des revendications 1 à 32, **caractérisée en ce que** la substance est injectée, dans un solvant approprié pouvant être supporté par le corps, localement dans la structure tissulaire du patient affectée par la douleur, ou bien est instillée localement sur la plaie opératoire, appliquée sur un nerf périphérique ou appliquée par voie transcutanée de façon appropriée.
